# EUROPEAN PATENT APPLICATION

(11) **EP 3 587 575 A1**
(43) Date of publication of application: **01.01.2020**
(21) Application number: 18305814.8
(22) Date of filing: 26.06.2018
(51) Int. Cl.: C12N 15/113, C12N 5/0775

(54) **MIRNA MIR-218 AND USE THEREOF FOR STIMULATING MESENCHYMAL STEM CELLS**

(71) Applicant: Institut National de la Santé et de la Recherche Médicale, 75013 Paris (FR); Centre Hospitalier Universitaire de Montpellier, 34295 Montpellier Cedex 05 (FR); Université de Montpellier, 34090 Montpellier (FR)
(72) Inventor: NOËL, Danièle, 34830 Clapiers (FR); JORGENSEN, Christian, 34980 Saint-Clément-de-Rivière (FR); PERS, Yves-Marie, 34090 Montpellier (FR); BONY-GARAYT, Claire, 34380 Saint-Martin-de-Londres (FR); DE VOS, John, 34270 Valfaunès (FR)
(74) Representative: LLR

(57) **Abstract**

The invention relates to the use of a composition comprising at least the miRNA miR-218, for stimulating, preferably in vitro, the immunosuppressive properties of mesenchymal stem cells, or MSCs.

## Description

The invention relates to mesenchymal stem cells and their use in therapy.

Mesenchymal Stem Cells (MSCs) are stromal cells present in a number of different tissue types. In addition to their ability to differentiate into multiple cell types of the mesenchyma, MSCs also display immunosuppressive properties. Whilst these mechanisms are far from fully understood, their immunosuppressive capacity has recently been shown to be modulated by microRNAs (miRNAs). The most extensively researched MSCs are those derived from bone marrow. However, these are difficult to isolate in high numbers, whilst adipose tissue-derived MSCs (ASCs) are much more accessible and have been shown to display similar properties. For this reason, their therapeutic potential for treatment of diseases with inflammatory responses, such as osteoarthritis, is of interest.

The invention therefore propose to provide a method for producing MSCs cells that can be used for their immunosuppressive properties.

Another aim of the invention is to provide compounds allowing to stimulate MSCs in order to enhance their putative immunosuppressive properties.

The present invention relates to the use of a composition comprising at least the miRNA miR-218 for stimulating, preferably *in vitro,* the immunosuppressive properties of multipotent stromal or mesenchymal stem cells, or MSCs.

The present invention is based on the unexpected observation made by the inventors that MSCs harbouring immunosuppressive properties express a high level of a specific miRNA, i.e. miR-218.

miR-218 microRNA precursor is a small non-coding RNA that regulates gene expression by antisense binding. miR-218 appears to be a vertebrate specific microRNA and has now been predicted and experimentally confirmed in a wide range of vertebrate species. The extents of the hairpin precursors are not known. In this case the mature sequence in excised from the 5'arm of the hairpin. miR-218, has been found to be silenced by DNA methylation in oral squamous cell carcinoma. It is also downregulated in Nasopharyngeal carcinoma, with artificially-induced expression serving to slow tumour growth. miR-218 has also been found to have tumour suppressing qualities in bladder cancer cells.

miR-218 is derived from the pre-miRNA sequence as depicted in SEQ ID NO : 4 : 
GUGAUAAUGUAGCGAGAUUUUCUGUUGUGCUUGAUCUAACCAUGUGGUU
GCGAGGUAUGAGUAAAACAUGGUUCCGUCAAGCACCAUGGAACGUCACGC
AGCUUUCUACA,
 wherein the sequence of miR-218 (SEQ ID NO : 1) is indicated in bold and the complementary antiparallel sequence (SEQ ID NO : 5) is represented as underlined.
The structure of the pre-miRN-218, as depicted in SEQ ID NO : 4 is the following one :

-    au   u   a    a    u      u          cu          gg    gag
5'-  gug   aa gu  gcg  gau  uucugu gugcuugau   aaccaugu   uugc    g
     |||   || ||  |||  |||  |||||| |||||||||   ||||||||   ||||    u
3'-  cau   uu cg  cgc  cug  aaggua cacgaacug   uugguaca   aaug    a
    a    cu   -   a    a    c      c          cc          -a    agu

Expression level of miR-218 can be enhanced by different ways, well known in the art. As demonstrated in the Example below, expression of miR-218 can be induced in ASCs by stimulation using cytokines such as IFNγ and TNFα.

Enhanced expression of miR-218 can be specifically obtained by using transfection protocols well known in the art such as transient or stable transfections.

When cells are transiently transfected, the miRNA is introduced into the cell as miRNA mimics but does not integrate into the chromosome. This means that many copies of the gene of interest are present, leading to high levels of molecules. Transfected miRNAs are effective in the cytoplasm, without the need to be transferred to the nucleus.

With stable or permanent transfection, miRNA can only be stably transfected when they are delivered as short hairpin transcripts made from a selectable DNA vector. However, RNA molecules per se cannot be used for stable transfection. By using specific combination of antibiotic resistance genes, cells having integrated, in their nuclear DNA, vectors containing the short hairpin transcripts sequences are selected and isolated from the non-transfected cells.

MiRNAs can be stably expressed as fragment of DNA encoding the miRNA of interest in the cell through lentivirus transduction.

For stimulation of expression of miR-218 in MSC, MSC can be isolated according to well-known protocols. For instance, but not limitative, ASC (Adipose Mesenchymal stem cells) can be isolated after enzymatic digestion of human fat tissue and culture as adherent cells from the stromal vascular fraction. These cells expressed CD34, CD90 and CD105 markers and are negative for CD31 and CD45.

According to the invention, MSC expressing miR-218 are immunosuppressive. This means that, in vitro and in vivo, MSCs inhibit the proliferation of virtually all immune cells, namely T cells, B-cells, natural killer cells (NK), monocytes/macrophages and dendritic cells (DC), producing what is known as division arrest anergy. They are also known to induce regulatory cells, such as Treg or Breg cells, that suppress immune responses. Moreover, immunosuppressive MSCs can stop a variety of immune cell functions: in particular but not exhaustively, cytokine secretion and cytotoxicity of T and NK cells; B cell maturation and antibody secretion; macrophage and DC maturation and activation; as well as antigen presentation.

Advantageously, the invention relates to the use as defined above, wherein said miR-218 comprises, consists essentially of, or consists of the nucleic acid sequence as set forth in SEQ ID NO: 1 (5'-AUGGUUCCGUCAAGCACCAUGG - 3').

The specific sequence of miRNA miR-218 is shown in SEQ ID NO: 1. When miR-218 is used by transfection, it can be advantageous to use either the nucleic acid molecule as set forth in SEQ ID NO: 4, or a complex comprising both sense and antisense sequence SEQ ID NO: 1 and SEQ ID NO: 5 , the complementarity being such that:

5'- ---UUGUGCUUGAUCUAACCAUGU -3' (SEQ ID NO: 1)
       | |||||||||  ||||||
3'- GGUACCACGAACUGCCUUGGUA-- -5' (SEQ ID NO: 5)

Advantageously, the invention relates to the use as defined above, wherein said miR-218 comprises, consists essentially of, or consists of the nucleic acid sequence as set forth in SEQ ID NO: 4.

Advantageously, the invention relates to the use as defined above, wherein said miR-218 comprises, consists essentially of, or consists of the complex of both nucleic acid molecules as set forth in SEQ ID NO: 1 and SEQ ID NO: 5.

More advantageously, the invention relates to the use as defined above, wherein said composition comprises or consists essentially of at least the miRNAs miR-218, miR-155 and miR-27B.

The inventors also made the unexpected observation that MSCs cells having a concomitant high expression of 3 miRNA, namely miR-218, miR-155 and miR-27B, harbors the immunosuppressive properties as defined above.

miR-155 is a microRNA that in humans is encoded by the MIR155 host gene or MIR155HG. MiR-155 plays an important role in various physiological and pathological processes. Exogenous molecular control *in vivo* of miR-155 expression may inhibit malignant growth, viral infections, and attenuate the progression of cardiovascular diseases. miR-155 is derived from the pre-miRNA sequence as depicted in SEQ ID NO : 6 : 
CUGUUAAUGCUAAUCGUGAUAGGGGUUUUUGCCUCCAACUGACUCCUAC
AUAUUAGCAUUAACAG,
 wherein the sequence of miR155 (SEQ ID NO : 2) is indicated in bold and the complementary antiparallel sequence (SEQ ID NO : 7) is represented as underlined.
The structure of the pre-miRN-155, as depicted in SEQ ID NO : 6 is the following one :

                c    a         -u   c
5'- ---uuaaugcuaau gug uagggguu  uug c
       ||||||||||| ||| ||||||||  |||
3'- gacaauuacgauua uac auccucag  aac u
                  -   -         uc   c

miR-27 is a family of microRNA precursors found in animals, including humans. MicroRNAs are typically transcribed as ∼70 nucleotide precursors and subsequently processed by the Dicer enzyme to give a -22 nucleotide product. The excised region or, mature product, of the miR-27 precursor is the microRNA mir-27. *Herpesvirus saimiri* expresses several non-coding RNAs (HSURs) which have been found to significantly reduce the level of miR-27 in a host cell. It has been proposed that miR-27 operates together with miR-23 and miR-24 in a cooperative cluster. miR-27B (also named hereafter miR-27b) is derived from the pre-miRNA sequence as depicted in SEQ ID NO : 8 : ACCUCUCUAACAAGGUGCAGAGCUUAGCUGAUUGGUGAACAGUGAUUGGU UUCCGCUUUG**UUCACAGUGGCUAAGUUCUGC**ACCUGAAGAGAAGGUG wherein the sequence of miR-27b (SEQ ID NO : 3) is indicated in bold and the complementary antiparallel sequence (SEQ ID NO : 9) is represented as underlined.
The structure of the pre-miRN-27b, as depicted in SEQ ID NO : 8 is the following one :

   -     -     aaca                    auug        ugau
5'-  accu cucu     aggugcagagcuuagcug      gugaacag      uggu
     |||| ||||     ||||||||||||||||||      ||||||||      ||| u
3'-  ugga gaga     uccacgucuugaaucggu      cacuuguu      gccu
    g     a    --ag                  --ga          --uc

Advantageously, the invention relates to the use as defined above, wherein said composition comprises miR-218 comprising the nucleic acid sequence as set forth in SEQ ID NO : 1, miR-155 comprising the nucleic acid sequence as set forth in SEQ ID NO : 2 (5'-UUGUGCUUGAUCUAACCAUGU-3') and miR-27B comprising the nucleic acid sequence as set forth in SEQ ID NO : 3 (5'-UUCACAGUGGCUAAGUUCUGC-3').

When miR-155 is used by transfection, it can be advantageous to use either the nucleic acid molecule as set forth in SEQ ID NO: 6, or a complex comprising both sense and antisense sequence SEQ ID NO: 2 and SEQ ID NO: 7, the complementarity being such that:

5'- CUGUUAAUGCUAAUCGUGAUAGGGUU -3' (SEQ ID NO: 2)
    |||||||||||||| ||| ||||||
3'- GACAAUUACGAUUA-UAC-AUCCUC-- -5' (SEQ ID NO: 7)

When miR-27b is used by transfection, it can be advantageous to use either the nucleic acid molecule as set forth in SEQ ID NO: 8, or a complex comprising both sense and antisense sequence SEQ ID NO: 3 and SEQ ID NO: 9, the complementarity being such that:

5'- AGGUGCAGAGCUUAGCUGAUUGGUGAAC -3' (SEQ ID NO: 9)
    ||||||||||||||||||   |||||
3'- UCCACGUCUUGAAUCGGU--GACACUU -5' (SEQ ID NO: 3)

Advantageously, the invention relates to the use as defined above, wherein said miR-218 comprises, consists essentially of, or consists of the nucleic acid sequence as set forth in SEQ ID NO: 4, miR-155 consists essentially of consists of the nucleic acid sequence as set forth in SEQ ID NO: 6, and miR-27B consists essentially of, or consists of the nucleic acid sequence as set forth in SEQ ID NO: 8.

Advantageously, the invention relates to the use as defined above, wherein said miR-218 comprises, consists essentially of, or consists of the complex of both nucleic acid molecules as set forth in SEQ ID NO: 1 and SEQ ID NO: 5, wherein said miR-155 comprises, consists essentially of, or consists of the complex of both nucleic acid molecules as set forth in SEQ ID NO: 2 and SEQ ID NO: 7, and wherein said miR-27B comprises, consists essentially of, or consists of the complex of both nucleic acid molecules as set forth in SEQ ID NO: 3 and SEQ ID NO: 9.

The invention also relates to an Isolated immunosuppressive MSC having an expression level of at least the miRNA miR-218 of at least 1.3 fold higher compared the expression level of the miR-218 of a non-immunosuppressive MSC.

The inventors identified that isolated MSC, i.e. MSC that are extracted from their natural context, and that are not present naturally in an individual, having a level of miR-218 which is 1.3 fold higher compared to non-immunosuppressive MSC harbor immunosuppressive properties.

The amount, or expression level, of miR-218 can be evaluated and measured by common technics well known in the art. For instance, real-time quantitative PCR (RQ-PCR) can be used.

The inventors have demonstrated that immunosuppressive ASCs, i.e. MSCs isolated from adipose tissues, have an expression level of miR-218 measured by RQ-PCR of at least 0.0170, whereas the miR-218 expression level measured by RQ-PCR is lower than 0.0126 for non-immunosuppressive ASCs.

Thus, advantageously, the invention relates to an Isolated immunosuppressive MSC having an expression level of at least the miRNA miR-218 of at least 0.0170 when measured by RQ-PCR.

Advantageously, the invention relates to an isolated immunosuppressive MSC as defined above, having an expression level of at least the miRNAs miR-218, miR-155 and miR-27B respectively 1.3 fold higher, 1.5 fold higher and 1.8 fold higher compared to the expression of miRNAs miR-218, miR-155 and miR-27B respectively of a non-immunosuppressive MSC.

The inventors also demonstrated that immunosuppressive ASCs, have an expression level of miR-218 measured by RQ-PCR of at least 0.0170, whereas the miR-218 expression level measured by RQ-PCR is lower than 0.0126 for non-immunosuppressive ASCs, have an expression level of miR-155 measured by RQ-PCR of at least 0.075, whereas the miR-155 expression level measured by RQ-PCR is lower than 0.047 for non-immunosuppressive ASCs, and have an expression level of miR-27B measured by RQ-PCR of at least 0.0046, whereas the miR-27B expression level measured by RQ-PCR is lower than 0.0025 for non-immunosuppressive ASCs.

Thus, advantageously, the invention relates to an Isolated immunosuppressive MSC having an expression level of the miRNA miR-218 of at least 0.0170, an expression level of the miRNA miR-155 of at least 0.075 and an expression level of the miRNA miR-27B of at least 0.0046, when measured by RQ-PCR

The invention further relates to a composition comprising, or comprising essentially, the miRNAs miR-218 comprising the nucleic acid sequence as set forth in SEQ ID NO : 1, miR-155 comprising the nucleic acid sequence as set forth in SEQ ID NO : 2 and miR-27B comprising the nucleic acid sequence as set forth in SEQ ID NO : 3.

The invention thus provides a composition comprising the 3 miRNA that are useful to induce immunosuppressive properties of the MSCs.

Advantageously, the invention relates to the composition as defined above comprising, or comprising essentially, the miRNAs miR-218 comprising the nucleic acid sequence as set forth in SEQ ID NO : 1 miR-155 comprising the nucleic acid sequence as set forth in SEQ ID NO : 2 and miR-27B comprising the nucleic acid sequence as set forth in SEQ ID NO : 3, each of the above nucleic acid molecule being in a form of salts, advantageously pharmaceutically acceptable salts such as sodium, calcium or potassium salts.

The above composition may also comprise a pharmaceutically acceptable vehicle well known in the art.

More advantageously the invention relates to the composition as defined above, wherein said miR-218 comprises, consists essentially of, or consists of the complex of both nucleic acid molecules as set forth in SEQ ID NO: 1 and SEQ ID NO: 5, wherein said miR-155 comprises, consists essentially of, or consists of the complex of both nucleic acid molecules as set forth in SEQ ID NO: 2 and SEQ ID NO: 7, and wherein said miR-27B comprises, consists essentially of, or consists of the complex of both nucleic acid molecules as set forth in SEQ ID NO: 3 and SEQ ID NO: 9, or advantageously a salt thereof.

More advantageously the invention relates to the composition as defined above, wherein said miR-218 comprises, consists essentially of, or consists of the complex of both nucleic acid molecules as set forth in SEQ ID NO: 4 and SEQ ID NO: 5, wherein said miR-155 comprises, consists essentially of, or consists of the complex of both nucleic acid molecules as set forth in SEQ ID NO: 6 and SEQ ID NO: 7, and wherein said miR-27B comprises, consists essentially of, or consists of the complex of both nucleic acid molecules as set forth in SEQ ID NO: 8 and SEQ ID NO: 9, or advantageously a salt thereof.

The invention also relates to a composition comprising a cell as defined above, for its use for the treatment of inflammatory pathologies or pathologies associated with inflammation.

This means that, *in vitro and in vivo,* MSCs inhibit the proliferation of virtually all immune cells, namely T cells, B-cells, natural killer cells (NK), monocytes/macrophages and dendritic cells (DC), producing what is known as division arrest anergy. They are also known to induce regulatory cells, such as Treg or Breg cells, that suppress immune responses. Moreover, immunosuppressive MSCs can stop a variety of immune cell functions: in particular but not exhaustively, cytokine secretion and cytotoxicity of T and NK cells; B cell maturation and antibody secretion; macrophage and DC maturation and activation; as well as antigen presentation.

The invention also relates to a composition for its use as defined above, wherein said pathologies associated with an inflammation are chosen from the group consisting of: osteoarticular diseases, inflammatory and autoimmune diseases.

Advantageously, the invention relates to the composition as defined above, for its use as defined above, wherein said osteoarticular diseases are osteoarthritis, arthritis and related musculoskeletal disorders and sclerodermias.

The invention also relates to a method for the *in vitro* stimulation of the immunosuppressive properties of isolated MSCs, said method comprising a step stimulating the expression of at least the miRNA miR-218.

According to the invention, the MSCs can be stimulated to become immunosuppressive by expressing of at least miR-218, in particular, miR-218 comprising, consisting essentially of, or consisting of the nucleic acid molecule as set forth in SEQ ID NO: 1, or miR-218 comprising, consisting essentially of, or consisting of the complex of both nucleic acid molecules as set forth in SEQ ID NO: 1 and SEQ ID NO: 5, or the pre miRNA miR-218 comprising, consisting essentially of, or consisting of the nucleic acid molecule as set forth in SEQ ID NO: 4.

In the method as defined above, MSCs previously obtained from an animal tissue, are cultures in an appropriated medium, and then are either stimulated by appropriated cytokines, such as IFNγ and/or TNFα, or other types of molecules (chemicals, peptides,...) or by transfection of at least one nucleic acid sequence coding for miR-218. miRNA can be transiently transfected as miRNA mimics or stably transfected as plasmid or after viral transduction.

After stimulation or transfection, MSCs that are stimulated are recovered and their immunosuppressive properties can be evaluated using classical *in vitro* assays, such as (but not exhaustive) T cell proliferation assays, mixed lymphocytes reactions, monocyte/macrophage or DC differentiation and maturation assays, NK cell cytotoxicity assays...

Advantageously, the invention relates to the method as defined above, wherein the step of stimulating the expression of at least the miRNA miR-218 is a step of ectopic expression of said at least miR-218.

More advantageously, the invention relates to the method as defined above, comprising a step stimulating the expression of the miRNA miR-218, miR-155 and miR-27b.

More advantageously, the invention relates to the method as defined above, comprising a step stimulating the expression of
- the miRNA miR-218 comprising, consisting essentially of, or consisting of the nucleic acid molecule as set forth in SEQ ID NO: 1, or miR-218 comprising, consisting essentially of, or consisting of the complex of both nucleic acid molecules as set forth in SEQ ID NO: 1 and SEQ ID NO: 5, or the pre miRNA miR-218 comprising, consisting essentially of, or consisting of the nucleic acid molecule as set forth in SEQ ID NO: 4,
- the miR-155 comprising, consisting essentially of, or consisting of the nucleic acid molecule as set forth in SEQ ID NO: 2, or miR-155 comprising, consisting essentially of, or consisting of the complex of both nucleic acid molecules as set forth in SEQ ID NO: 2 and SEQ ID NO: 7, or the pre miRNA miR-155 comprising, consisting essentially of, or consisting of the nucleic acid molecule as set forth in SEQ ID NO: 6, and
- the miR-27b comprising, consisting essentially of, or consisting of the nucleic acid molecule as set forth in SEQ ID NO: 3, or miR-155 comprising, consisting essentially of, or consisting of the complex of both nucleic acid molecules as set forth in SEQ ID NO: 3 and SEQ ID NO: 9, or the pre miRNA miR-27b comprising, consisting essentially of, or consisting of the nucleic acid molecule as set forth in SEQ ID NO: 8.

The invention also relates to a composition or a kit comprising at least the miRNA miR-218 and at least one isolated MSC.

More advantageously, the invention relates to the above composition or kit comprising at least the miRNA miR-218 comprising, consisting essentially of, or consisting of the nucleic acid molecule as set forth in SEQ ID NO: 1, or at least miR-218 comprising, consisting essentially of, or consisting of the complex of both nucleic acid molecules as set forth in SEQ ID NO: 1 and SEQ ID NO: 5, or at least the pre miRNA miR-218 comprising, consisting essentially of, or consisting of the nucleic acid molecule as set forth in SEQ ID NO: 4, and
at least one isolated MSC.

The invention advantageously relates to the above-mentioned kit comprising the miRNAs miR-218, miR-155 and miR-27B, and at least one isolated MSC.

More advantageously, the invention relates to the above mentioned composition or kit, comprising
- the miRNA miR-218 comprising, consisting essentially of, or consisting of the nucleic acid molecule as set forth in SEQ ID NO: 1, or miR-218 comprising, consisting essentially of, or consisting of the complex of both nucleic acid molecules as set forth in SEQ ID NO: 1 and SEQ ID NO: 5, or the pre miRNA miR-218 comprising, consisting essentially of, or consisting of the nucleic acid molecule as set forth in SEQ ID NO: 4,
- the miR-155 comprising, consisting essentially of, or consisting of the nucleic acid molecule as set forth in SEQ ID NO: 2, or miR-155 comprising, consisting essentially of, or consisting of the complex of both nucleic acid molecules as set forth in SEQ ID NO: 2 and SEQ ID NO: 7, or the pre miRNA miR-155 comprising, consisting essentially of, or consisting of the nucleic acid molecule as set forth in SEQ ID NO: 6, and
- the miR-27b comprising, consisting essentially of, or consisting of the nucleic acid molecule as set forth in SEQ ID NO: 3, or miR-27b comprising, consisting essentially of, or consisting of the complex of both nucleic acid molecules as set forth in SEQ ID NO: 3 and SEQ ID NO: 9, or the pre miRNA miR-27b comprising, consisting essentially of, or consisting of the nucleic acid molecule as set forth in SEQ ID NO: 8,
and at least one MSC.

The invention will be better understood in light of the following 10 figures and the following example.

### LEGENDE TO THE FIGURES

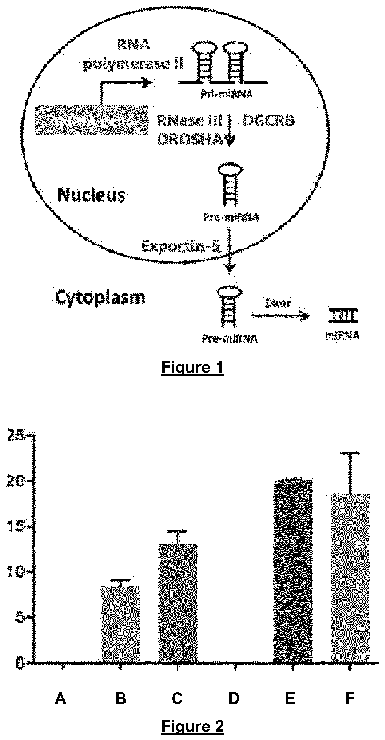

**Figure 1** is a Visual representation to summarise miRNA biogenesis. Figure adapted from Goodall et al. (2013).
**Figure 2** is a graph showing mean expression of IDO1 for MSC1 and MSC2 under different stimulation conditions. From left to right: MSCs incubated with unsupplemented media (A), media supplemented with 10ng/mL IFNγ (B), media supplemented with 20ng/mL IFNγ (C), media supplemented with 10ng/mL TNFα (D), media supplemented with 10ng/mL TNFα and 10ng/mL IFNγ (E), and media supplemented with 10ng/mL TNFα and 20ng/mL IFNγ (F). Results are expressed as IDO1 expression normalised (2^{-ΔCt}) to RSP9.

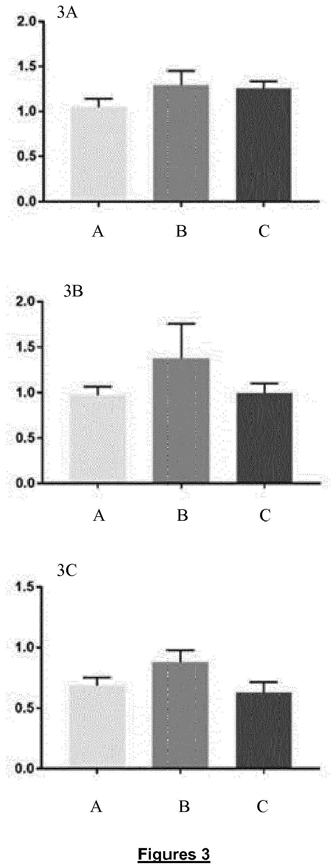

**Figures 3A to 3C** are graphs showing Preliminary Expression Profiles for (3A to 3C) miR-146a, 24 hours (3A), 48 hours (3B) or 72 hours (3C) following stimulation with inflammatory cytokines (B : 20ng/mL IFNγ; C: 20ng/mL IFNγ and 10ng/mL TNFα). Graphs display average miRNA expression for 6 ASCs normalised (2^{-ΔCt}) to RNU48 expression with SEM error bars.

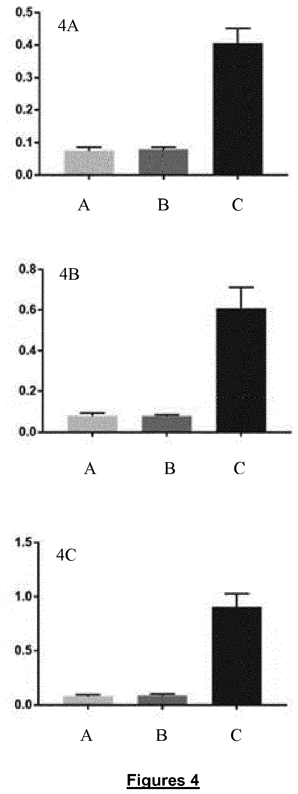

**Figures 4A to 4C** are graphs showing Preliminary Expression Profiles for (3A to 4C) miR-155, 24 hours (4A), 48 hours (4B) or 72 hours (4C) following stimulation with inflammatory cytokines (B : 20ng/mL IFNγ; C: 20ng/mL IFNγ and 10ng/mL TNFα). Graphs display average miRNA expression for 6 ASCs normalised (2^{-ΔCt}) to RNU48 expression with SEM error bars.

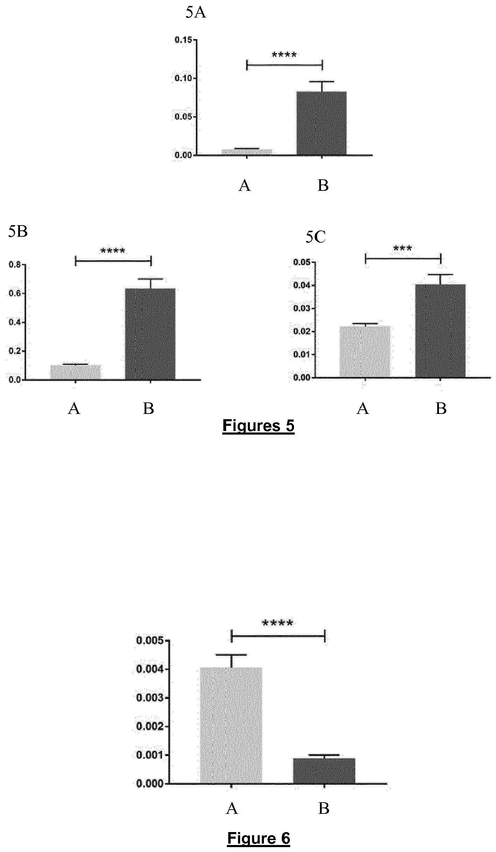

**Figures 5A to 5C** represent graphs showing Expression profiles for miRNAs that were upregulated upon stimulation: 5A) miR-146a, 5B) miR-155, 5C) miR-218. This upregulation is significant for all miRNAs. Non-stimulated ASCs (A) have been incubated in unsupplemented media for 48 hours. Stimulated ASCs (B) have been incubated in media supplemented with 20ng/mL IFNγ and 10ng/mL TNFα. Graphs display average miRNA expression for 15 ASCs normalized (2^{-ΔCt}) to RNU48 expression, with SEM error bars.
**Figure 6** represents a graph showing Expression profiles for miRNA miR-27b that were significantly downregulated upon stimulation. Non-stimulated ASCs (A) have been incubated in unsupplemented media for 48 hours. Stimulated ASCs (B) have been incubated in media supplemented with 20ng/mL IFNγ and 10ng/mL TNFα. Graphs display average miRNA expression for 15 ASCs normalised (2^{-ΔCt}) to RNU48 expression, with SEM error bars.

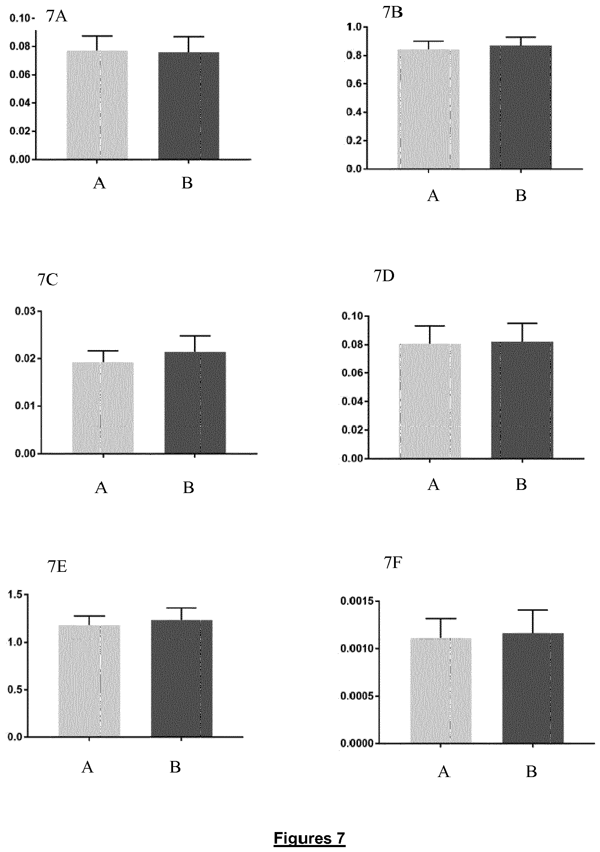

**Figures 7A to 7F** represent graphs showing Expression profiles for miRNAs whose expression was not significantly changed by stimulation: 7A) miR-23b, 7B) miR-29a, 7C) miR-29b, 7D) miR-29c, 7E) miR-149 and 7F) miR-545. Non-stimulated ASCs (A) have been incubated in unsupplemented media for 48 hours. Stimulated ASCs (B) have been incubated in media supplemented with 20ng/mL IFNγ and 10ng/mL TNFα. Graphs display average miRNA expression for 15 ASCs normalised (2^{-ΔCt}) to RNU48 expression, with SEM error bars.

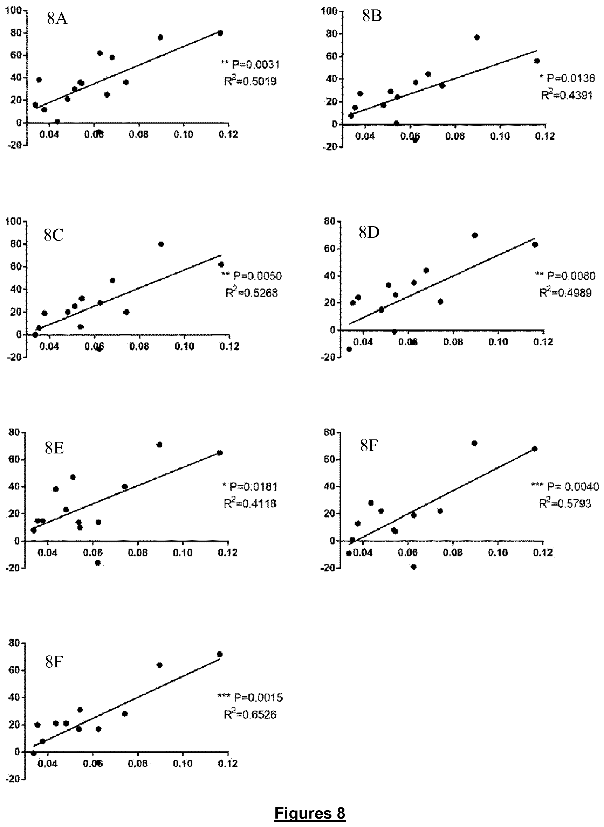

**Figures 8A-8G** represents graphs showing Linear Regression showing pain and function analyses as a function of miR-155 expression in patients where results are significant. Expression of miR-155 has been normalised (2^{-ΔCt}) to RNU48 expression. The R² value measuring the goodness of fit, and the P value measuring the significance of the deviation of the slope from zero are displayed next to each graph.
Figure 8A represent a graph showing mir-155 expression correlation with Δ VAS at 3 months.
Figure 8B represent a graph showing mir-155 expression correlation with Δ WOMAC Total at 3 months.
Figure 8C represent a graph showing mir-155 expression correlation with Δ WOMAC Pain at 3 months.
Figure 8D represent a graph showing mir-155 expression correlation with Δ WOMAC Function at 3 months.
Figure 8E represent a graph showing mir-155 expression correlation with Δ WOMAC Total at 6 months.
Figure 8F represent a graph showing mir-155 expression correlation with Δ WOMAC Pain at 6 months.
Figure 8G represent a graph showing mir-155 expression correlation with Δ WOMAC Function at 6 months.
**Figures 9A to 9G** represent graph showing Linear Regression showing pain and function analyses as a function of miR-218 expression in patients where results are significant. Expression of miR-218 has been normalised (2^{-ΔCt}) to RNU48 expression. The R2 value measuring the goodness of fit, and the P value measuring the significance of the deviation of the slope from zero are displayed next to each graph.

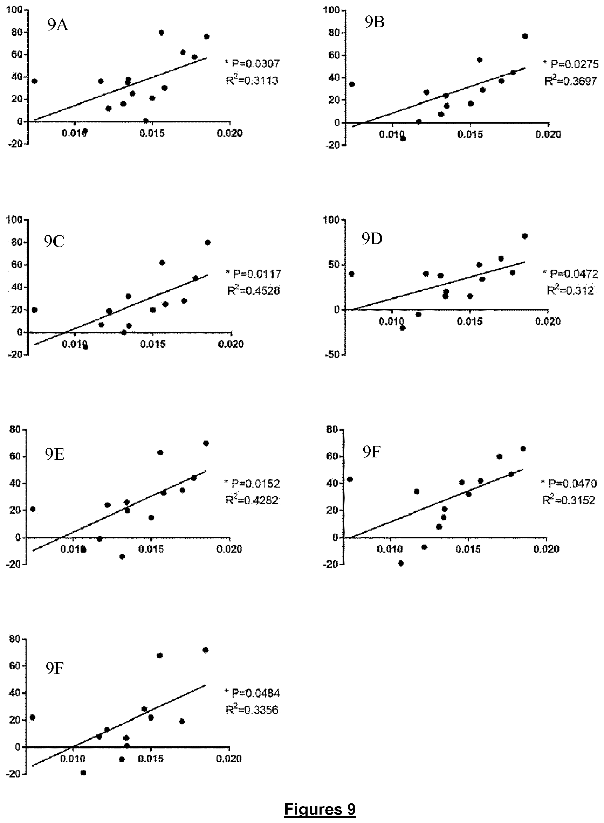

Figure 9A represent a graph showing mir-218 expression correlation with Δ VAS at 3 months.
Figure 9B represent a graph showing mir-218 expression correlation with Δ WOMAC Total at 3 months.
Figure 9C represent a graph showing mir-218 expression correlation with Δ WOMAC Pain at 3 months.
Figure 9D represent a graph showing mir-218 expression correlation with Δ WOMAC Function at 3 months.
Figure 9E represent a graph showing mir-218 expression correlation with Δ WOMAC Total at 6 months.
Figure 9F represent a graph showing mir-218 expression correlation with Δ WOMAC Pain at 6 months.
Figure 9G represent a graph showing mir-218 expression correlation with Δ WOMAC Function at 6 months.

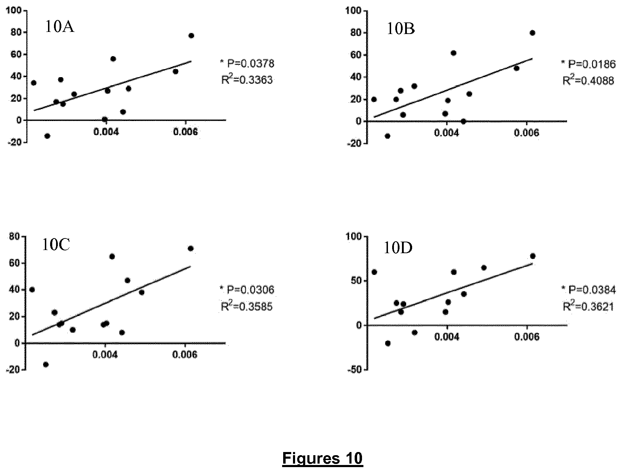

**Figures 10A to 10D** represent graphs showing Linear Regression showing pain and function analyses as a function of miR-27b expression in patients where results are significant. Expression of miR-27b has been normalised (2^{-ΔCt}) to RNU48 expression. The R2 value measuring the goodness of fit, and the P value measuring the significance of the deviation of the slope from zero are displayed next to each graph.
Figure 10A represent a graph showing mir-27b expression correlation with Δ WOMAC Total at 3 months.
Figure 10B represent a graph showing mir-27b expression correlation with Δ WOMAC Pain at 3 months.
Figure 10C represent a graph showing mir-27b expression correlation with Δ WOMAC Total at 6 months.
Figure 10D represent a graph showing mir-27b expression correlation with Δ WOMAC Stiffness at 6 months.

### EXAMPLE

### Introduction

Mesenchymal Stem Cells (MSCs) are nonhematopoietic stromal cells that are able to give rise to a number of differentiated cell types. These multipotent cells can differentiate into various cells present in the mesenchyma, including osteoblasts, chondrocytes, adipocytes among other cell types. In addition, according to the International Society for Cellular Therapy (I.S.C.T), MSCs are capable of self-renewal and can be defined by expression and lack of expression of specific surface antigens, as shown in Table 1, which distinguish them from hematopoetic cells. Furthermore these cells adhere to plastic when cultured, where they have a fibroblast-like phenotype, and have the ability to develop fibroblast colony-forming-units.

### Table 1

**Table 1: Summary of specific surface antigen phenotype necessary to define a MSC.**

| Positive (≥95% of MSC population) | Negative (≤2% of MSC population) |
|---|---|
| • CD73 | • CD11b or CD14 |
| • CD90 | • CD19 or CD79α |
| • CD105 | • CD34 |
| | • CD45 |
| | • Human leukocyte antigen-DR |

MSCs were first identified in bone marrow but have since been found to reside in many other tissue types, including adipose tissue and may be present in nearly all tissues. For this reason, most studies of MSCs have been conducted on those found in bone marrow (BM-MSCs), though these share many similarities with adipose tissue-derived MSCs (ASCs). Both have fibroblast-like cell morphology, and express the mesenchymal markers CD73 and CD90, however markers CD34 and CD106 are differentially expressed: the two cell lines have distinct phenotypic profiles. Additionally, Noel et al. observed further cell-specific differences at mRNA, proteomic and functional levels. Moreover, ASCs have also become a more attractive therapeutic target, as they are more accessible and can be isolated in greater quantities.

In addition to their differentiation capacities, MSCs have paracrine effects as well as immunomodulatory capabilities. In order for MSCs to become immunosuppressive, they must be preliminarily activated by inflammatory cytokines (secreted by immune cells): mainly interferon γ (IFNγ) and potentially in concert with Tumour Necrosis Factor α (TNFα), Interleukin-1α (IL-1α) or Interleukin-1β (IL-1β). IFNγ stimulation notably results in activation of indoleamine 2,3-dioxygenase (IDO1), which subsequently metabolises tryptophan to kynurenine. The subsequent local accumulation of tryptophan metabolites results in immunosuppression (Ryan et al., 2007). Moreover, activated MSCs inhibit the proliferation and function of all immune cells; this has been extensively reviewed in the art.

More recently, microRNAs (miRNAs) have been the subject of interest for many areas of research, and have been increasingly implicated in modulation of the immunosuppressive effect of MSCs. miRNAs are small noncoding single-stranded RNAs of ∼22 nucleotides (nt) in length, initially discovered in C. elegans. In humans, miRNAs are primarily transcribed from introns and exons by RNA polymerase II to form primary miRNAs of approximately 200-4000 nt (Lee et al., 2004). These have a 5' 7-methylguanosine cap, a 3' poly(A) tail and fold into imperfectly-paired hairpin structures. In the nucleus, RNase III DROSHA and its cofactor DiGeorge Syndrome Critical Region Gene 8 (DGCR8) catalyse the first excision to produce the pre-miRNA molecule: a 70-100nt hairpin with a monophosphate 5' terminus and a hydroxyl group at the 3' terminus. Exportin-5 then mediates the export of pre-miRNAs to the cytoplasm, where the RNase III enzyme Dicer along with transactivating response RNA binding protein carry out further processing to produce the 20-22nt double-stranded mature miRNA. Dicer then initiates the formation of the RNA-induced silencing complex (RISC), which contains members of the Argonaute family. This protein is partially responsible for the selection of the single stranded miRNA that associates with RISC to become active miRNA. A summary of this process can be seen in **Figure 1****.** The complex then associates with 3' or 5'-untranslated regions of target mRNAs by imperfect complementary base-pair matching, leading to inhibition of gene expression via chromatin modification and silencing, mRNA degradation or translational repression.

It is in this way that miRNAs have become increasingly reported to play a role in modulating immunosuppression by MSCs. For example, authors demonstrated that upon activation of murine MSCs by IFNγ (with TNFα or IL-1α), miR-155 is significantly upregulated. miR-155 targets TAK1-binding protein 2, which reduces its expression at both mRNA and protein levels. This in turn results in decreased expression of inducible nitric oxide synthase (iNOS) and thus inhibits the immunosuppressive capability of MSCs. miR-181a has been shown to decrease MSC ability to inhibit T-cell proliferation in vivo and in vitro, thus attenuating their immunosuppressive abilities. Further examples of miRNAs modulating the immunosuppressive effects of MSCs have been described previously. Additionally, previous analysis by the group identified certain miRNAs as being modified in BM-MSCs upon priming by activated PBMCs. Of 754 miRNAs tested in a Taqman Array Card (Life Technologies), 16 were found to be significantly modulated in primed BM-MSCs by at least a 2 fold ratio.

In light of this immunosuppression, research has also been directed to inflammatory rheumatic diseases, of which osteoarthritis (OA) is the most common. OA is characterized by abnormal remodeling and degradation of joint tissue, and this degradation is further exacerbated by inflammation. This results in degeneration of articular cartilage and sclerosis of subchondral bone as well as synovial inflammation. Toll-like receptors expressed by chondrocytes (cells that are located in cartilage and involved in cartilage homeostasis) are activated by damage-associated molecular patterns. This activation induces chondrocytes to undergo metabolic modifications favoring catabolism, and thus the cartilage degradation associated with OA. This degradation further contributes to the remodeling of subchondral bone and ossification. The chondrocytes and osteoblasts implicated in these two processes also play a role in synovial inflammation, by releasing inflammatory mediators that drive joint degeneration - a positive feedback cycle.

Recent research has shown miRNAs to be deregulated in OA. For example, miR-140 is downregulated in OA cartilage in comparison to normal tissue, similar to miR-146 which is also downregulated. In addition, increased expression of inflammatory cytokines in human OA cartilage is associated with decreased miR-149 expression. Further examples have been more extensively reviewed by the inventors. Whilst this research has increased scientific understanding of the disease, no treatment currently impacts upon the progressive degeneration of joint tissues. For this reason, the lab has previously conducted research and clinical trials to investigate the therapeutic use of MSCs. The clinical trial featured 18 patients with symptomatic and severe primary knee OA, and aimed to evaluate the safety of a singular intra-articular injection of autologous ASCs in a phase I study, with a vision to develop this as a therapeutic treatment. Safety was demonstrated and significant improvements in pain levels and function were seen; these promising results led to a phase II study which is currently taking place.

In the present study, the link between miRNA expression and the therapeutic potential of MSCs in OA was investigated. Firstly the inventors aimed to determine the role of ASC activation by inflammatory cytokines IFNγ and TNFα on modulating the expression of miRNAs, and thus further understand how this may contribute to the immunosuppressive effect of MSCs. These ASCs were isolated from patients during the clinical trial described above, and thus can be used to develop a better understanding of how the cells exerted their therapeutic immunosuppressive effects in vivo. Secondly, the study investigated the prognostic value of miRNA expression by ASCs for therapeutic efficacy in the treatment of osteoarthritis. Here the inventors compared the miRNA expression profiles of these ASCs with clinical data to determine if there is any correlation between ASC miRNA expression and the therapeutic efficacy of the ASC injection into an OA joint, with a perspective to ultimately create a prognostic profile that could better predict the success of an autologous ASC injection to treat OA.

### Materials and Methods

### MSC Cell Culture

Two BM-MSC samples that the lab had previously extracted from human patients and frozen down were defrosted. The cells were resuspended in α-MEM medium (Lonza) supplemented with 10% Foetal Calf Serum (Invitrogen), 100U/mL Penicillin/Streptomycin (Lonza), 2mM L-Glutamine (Lonza) and 1ng/mL basic fibroblast growth factor (bFGF; Cellgenix), and seeded at a density of 100,000 cells per well in 6-well plates. The cells were cultured at 37°C in a 5% CO2 incubator. After 24 hours in culture, cells were washed in Phosphate-Buffered Saline (PBS) and differentially stimulated in 2mL medium supplemented with one of the following: unsupplemented medium, medium with 10ng/mL IFNγ, medium with 20ng/mL IFNγ, medium with 10ng/mL TNFα, medium with 10ng/mL IFNγ and 10ng/mL TNFα, or medium with 20ng/mL IFNγ and 10ng/mL TNFα (as shown in Table 2). 24 hours after stimulation, cells were trypsinized and centrifuged at room temperature at 400G for 5 minutes, washed in PBS, and the pellet was then resuspended in 700µL Qiazol lysis buffer for RNA extraction (Qiagen) before the sample was frozen at -80°C.

**Table 2**

| Negative Control | 10ng/mL IFNγ | 20ng/mL IFNγ |
|---|---|---|
| 10ng/mL TNFα | 10ng/mL IFNγ | 20ng/mL IFNγ |
| | 10ng/mL TNFα | 10ng/mL TNFα |

**Table 2 :** Visual demonstration of stimulation conditions in 6-well plates for both BM-MSCs. Top row left to right : BM-MSCs in media alone, medium with 10ng/mL IFNγ, medium with 20ng/mL IFNγ. Bottom row left to right medium with 10ng/mL TNFα, medium with 10ng/mL IFNγ and 10ng/mL TNFα, medium with 20ng/mL IFNγ and 10ng/mL TNFα.

### ASC Cell Culture

Human ASC samples from patients having previously taken part in a clinical trial conducted by the lab (Pers et al., 2016) were defrosted. The cells were resuspended in supplemented α-MEM medium as detailed above and seeded at 60,000 cells per well in 6-well plates in 2mL media. "Non-cultured" cells were harvested in trypsin upon adhesion to the bottom of the well, which occurred approximately 24 hours after being plated. These cells were subsequently washed in PBS and resuspended in 700µL Qiazol, and then frozen at -80°C. 24 hours after plating, media was removed from cultured cells, which were then stimulated in either i) media supplemented with 20ng/mL IFNγ, ii) media supplemented with 20ng/mL IFNγ and 10ng/mL TNFα, or iii) unsupplemented fresh media. These cells were subsequently harvested in Qiazol as described above 24 hours, 48 hours and 72 hours after stimulation.

### RNA Extraction

Total RNA (including small RNAs such as miRNAs) was extracted using the miRNeasy Micro Kit (Qiagen) following the manufacturer's protocol. Cells were lysed with Qiazol and by passage through a syringe and 20 gauge needle tip. DNA and proteins were separated from RNA by addition of chloroform and centrifugation at 15000G for 15 minutes at 4°C. The upper aqueous phase containing RNA was collected and RNA was extracted using miRNeasy spin columns, 80% ethanol and RWT and RPE buffers, using an automated QiaCube machine (Qiagen). The RNA was eluted with DNase-RNase-free water. Total RNA quantity was measured using a Nanodrop ® ND-1000 spectrophotometer, and the RNA extraction quality verified using 260:280 and 260:230 ratios. RNA was stored at -80°C.

### Reverse Transcription

To quantify mRNA expression, total RNA was reverse transcribed using an Invitrogen Kit (Thermofisher) following the manufacturer's protocol. RNA extractions prepared as described above were diluted in DNase and RNase-free water to give 400ng of RNA for each cDNA synthesis reaction. These dilutions were incubated in the thermocycler (Eppendorf ® Mastercycler) with 2.5µM Random Hexamers and 500µM dNTP mix for 5 minutes at 65°C. Addition of 1X M-MLV-TR buffer, 10mM dithiothreitol and 100 U M-MLV Reverse Transcriptase was carried out on ice. Samples were transferred back to the thermocycler, and reverse transcription programme was run following the manufacturer's protocol: 10 minutes at 25°C; 50 minutes at 37°C; 15 minutes at 70°C. The cDNA produced was stored at -20°C.

To quantify miRNA expression, specific reverse transcription of miRNAs was carried out using a TaqMan MicroRNA Reverse Transcription Kit (Applied Biosystems) following the manufacturer's protocol, in which small RNA-specific stem loop RT primers are used to produce cDNA. A mix of 1X RT Buffer, 2.5mM dNTP mix, 3.8 Units RNase Inhibitor, 150 Units Multiscribe RT and 0.025X Taqman miRNA RT primer was added to 150ng RNA, briefly centrifuged and incubated on ice for 5 minutes. Samples were transferred to the thermocycler and the programme for miRNA reverse transcription was carried out following the manufacturer's protocol (16°C for 30 minutes; 42°C for 30 minutes; 85°C for 5 minutes). Samples were stored at -20°C.

### Real Time PCR

To quantify gene expression, Real Time Quantitative Polymerase Chain Reaction (RT-qPCR) was performed with 0.25 µM of each forward and reverse primers, 1X LightCycler ® 480 SYBR Green I Master and 20ng cDNA (transcribed from total RNA) per PCR reaction. The following programme was run using a ViiA™7 machine (Applied Biosystems): 50°C for 2 minutes, 95°C for 10 minutes, 40 cycles of 95°C for 15 seconds; 64°C for 1 minute, 95°C for 15 seconds and 60°C for 1 minute. Analysis of mRNA expression level was performed using ViiA™7 software v1.2.2. Expression levels were normalised to the expression of Ribosomal Protein S9 (RPS9) mRNA using the formula 2-(ΔCT).

To quantify miRNA expression, RT-qPCR was performed using 1X TaqMan ® Universal PCR Master Mix (no UNG), 1X TaqMan ® miRNA TM primer, and 5ng miRNA specific cDNA per reaction. The following program was run: 50°C for 2 minutes, 95°C for 10 minutes, 40 cycles of 95°C for 15 seconds; 60°C for 1 minute, 95°C for 15 seconds and 60°C for 1 minute. Levels of miRNA expression were normalised to expression of RNU48 (a miRNA control).

### Statistical Analysis

GraphPad Prism software was used for statistical analysis and graphical representation. P values were used to determine significance of results where *P ≤ 0.05, **P ≤ 0.01, ***P ≤ 0.001, ****P ≤ 0.0001.

### Clinical Data

The lab previously conducted a phase I clinical trial (Pers et al., 2016) to evaluate the safety and clinical efficacy of injecting autologous ASCs into patients with knee OA. There were 18 participants in the trial, all of which had similar baseline characteristics for age, sex and body mass index. For each patient, two samples of ASCs were prepared from adipose tissue extracted by liposuction: one of these samples was used for the intra-articular injection, and where the second sample (which was prepared as a back-up sample) was unused it was immediately frozen in liquid nitrogen. After follow-up consultations at 0 months, 3 months and 6 months following the injection, the procedure was found to be safe, and no patients reported serious adverse side-effects. With regards to evaluation of clinical efficacy, pain and function subscales including the pain visual analog scale (VAS) and the Western Ontario and McMAster Universities Arthritis Index (WOMAC) were used. A 0- to 100-mm visual analog scale was used to assess scores for WOMAC pain (5 questions), WOMAC physical function (17 questions) and WOMAC stiffness (2 questions), with an overall WOMAC total score also calculated. Scores obtained 3-months and 6-months after the injection were subtracted from baseline statistics to give (M0-M3) or (M0-M6) values, as a measure of the change in pain/function following the procedure. The values calculated for each patient were then compared with the miRNA expression of the corresponding "non-cultured" ASCs using linear regression analysis and GraphPad Prism.

### Results

### Validation of Cytokines: Concentration and Efficacy

MSCs isolated from bone marrow in human patients (MSC1, MSC2) were grown in culture and plated at 100,000 cells in 6-well plates. To verify that the stocks of IFNγ and TNFα were still potent, the MSCs were incubated in varying concentrations of IFNγ with and without TNFα. 24 hours after stimulation with these cytokines, the cells were harvested and the RNA was extracted and reverse transcribed. RT-qPCR was carried out for IDO1, which is induced by IFNγ, and this was normalised against RSP9. As shown in **Figure 2****,** the results demonstrated an upregulation of IDO1 in the presence of IFNγ, validating the IFNγ efficacy. This upregulation was enhanced when in concert with TNFα. This confirmed that subsequent experiments could be carried out using the stock of cytokines at these concentrations.

### Optimisation of Conditions to Activate ASCs

In order to identify the best conditions and time point at which to harvest cells, 6 ASCs (out of the 15 that were available to the group) that had previously been isolated from patients by liposuction (Pers et al., 2016) were initially grown in culture. miRNA expression profiles for 2 miRNAs known to be modulated in BM-MSCs upon activation by PBMCs (miR-146a, miR-155) were compared for ASCs following 24, 48 or 72 hours of growth in either unsupplemented media, media supplemented with 20ng/mL IFNγ, or media supplemented with 20ng/mL IFNγ and 10ng/mL TNFα. These miRNAs were assayed as they had previously been identified as of interest to the lab from previous research, or were cited in relevant literature as being implicated in the immunomodulatory effect of MSCs. As can be seen in **Figures 3** and **Figures 4****,** the changes in expression between stimulated and unstimulated cells was generally greatest when the ASCs had been stimulated by both IFNγ and TNFα. In addition, the combination of IFNγ and TNFα more closely represents in vivo conditions. Given these results, it was decided for subsequent experiments to reduce the stimulation conditions to unsupplemented media and media supplemented by both 20ng/mL IFNγ and 10ng/mL TNFα. Furthermore, the expression profiles for ASCs at 48 hours and 72 hours did not differ greatly. Harvesting cells at 48 hours instead of 72 hours more effectively avoids any effects of cells becoming over-confluent in culture on miRNA expression, so it was decided that 48 hours after stimulation would be the only time point at which cells would be harvested.

### RT-qPCR Analysis of miRNA Expression in Patient ASCs

Following optimisation of stimulation conditions, the final 9 ASCs (to total 15) were defrosted and grown in culture to analyze expression profiles for a wider range of miRNAs. Again, ASCs were stimulated 24 hours after plating, and were harvested 48 hours following cytokine stimulation. Reverse Transcription for specific miRNAs was carried out for all 15 ASCs in stimulated and non-stimulated conditions. In addition to the 2 that were initially assayed, RT-qPCR was then carried out the following miRNAs: miR-23b, miR-27b, miR-29a, miR-29b, miR-29c, miR-149, miR-218 and miR-545.The miRNAs assayed were chosen based on a previous analysis run by the lab (see introduction) or from data in the literature.

The miRNA expression profiles in **Figures 5 to** 7 show the variation in expression between stimulated and non-stimulated ASCs. In **Figures 5** it can be seen that upon stimulation with inflammatory cytokines, expression of miR-146a, miR-155, and miR-218 significantly increases in ASCs. Conversely, in **Figure 6** a significant decrease in expression of miR-27b can be seen. Expression of miR23b, miR-29a, miR-29b, miR-29c, miR-149, and miR-545 are not significantly changed; this can be seen in **Figures 7****.**

### Comparison of miRNA Expression Profiles with Clinical Data

The inventors previously ran a clinical trial which showed that an injection of autologous patient ASCs into an osteoarthritic joint resulted in significant reductions in pain and improved function (as compared to levels before the injection). Different subscales including VAS and WOMAC scores were used to evaluate levels of pain and function, and thus the efficacy of the autologous injection in patients. A 0- to 100-mm visual analog scale (going from high to low pain/low to high function) was used to assess WOMAC pain, WOMAC stiffness, and WOMAC physical function, with an overall WOMAC total score also calculated. A breakdown of subject matter analysed for each scale can be seen in Table 3. Scores obtained 3 months and 6 months after the injection were subtracted from baseline values to give M0-M3 (3 months) or M0-M6 (6 months) values. These values were correlated with miRNA expression for ASCs using GraphPad Prism.

### Table 3

**Table 3: Breakdown of items used to assess the three WOMAC subscales.**

| | |
|---|---|
| **Pain** (5 items) | During walking, using stairs, in bed, sitting or lying, and standing |
| **Stiffness** (2 items) | After first walking, and later in the day |
| **Physical Function** (17 items) | Stair use, rising from sitting, standing, bending, walking, getting in/out of a car, shopping, putting on/taking off socks, rising from bed, lying in bed, getting in/out of the bath, sitting, getting on/off the toilet, heavy household duties, light household duties |

Levels of expression for three miRNAs were identified as being significantly correlated with improvements in pain/function: miR-155, miR-218 and miR-27B. For all three miRNAs, a higher level of miRNA expression in cells correlates with a better pain/function improvement both 3 months and 6 months after ASC injection into the OA joint (**Figures 8-10**).

### Discussion

Here the inventors report significant changes in miRNA expression profiles in ASCs, upon induction of the cells to become immunosuppressive by the inflammatory cytokines IFNγ and TNFα. Of the miRNAs assayed, it can be seen in **Figures 5** that miR-146a, miR-155, miR-218 expression significantly increased whilst expression of miR-27b is significantly decreased (**Figure 6**).The changes in expression of miR-146a and miR-155 upon ASC activation correlate with the lab's unpublished data regarding the expression of these miRNAs in activated BM-MSCs. That said, it is worth noting that whilst in both instances the MSCs were induced to become immunosuppressive in inflammatory conditions, the BM-MSCs were activated by activated peripheral blood mononuclear cells (PBMCs) whilst the ASCs were activated by inflammatory cytokines. The other miRNAs have not been extensively researched by the lab as of yet. However, as research has increasingly become focused on miRNAs in recent years, more information is available in the literature to help elucidation of the specific role(s) each miRNA plays in MSCs. miR-218 was identified by Hu et al. (2017) to regulate neuronal differentiation of ASCs through the Wnt signalling pathway, whilst Chen et al. (2013) identified miR-27b as a signature for ASCs involved in the tolerogenic response. As this research continues, it will be possible to further compare the expression levels of miRNAs under different conditions, including when they are induced to become immunosuppressive. This will enable us to better contextualise the inventors' results and to understand their significance on a larger scale. However, the result of changed miRNA expression on MSCs is not always clear or as anticipated. Expression of miR-155 is increased in inflammatory conditions, but this inhibits the immunosuppressive effect of MSCs by reducing expression of the iNOS enzyme. Whilst this does not immediately seem logical, this could suggest a regulatory role for miR-155 (such as in feedback inhibition) - further research in this area could confirm this. In addition, it will be interesting to investigate the relationship between the change in expression of other miRNAs and the immunomodulation of MSCs - does the modulated expression result in MSCs becoming immunosuppressive, or does the immunosuppression itself modulate miRNA expression levels? Upon elucidation of the pathways involving miRNAs and MSC immunosuppression some of these key questions will begin to be answered.

Nevertheless, this study is preliminary and the results have not been repeated. However, if repeats on the same cell lines were carried out, the freeze-thaw cycles associated with culturing the cells may modify their miRNA expression profiles. Decreased cell proliferation was observed for cells cultured with the inflammatory cytokines in comparison with those cultured in unsupplemented media. Increased cell mortality associated with decreased cell proliferation may have had a modulating effect on the expression of miRNAs, particularly due to secretion of factors by cells upon cell death. In addition, only one inflammatory condition was tested here. Testing the reproducibility of the inventors' results using different inflammatory conditions would strengthen their reliability and prove that the effects seen are not exclusive to activation by IFNγ and TNFα.

Secondly, the study demonstrated significant correlations between improved pain/function following injection of autologous ASCs into an OA joint with expression levels of miR-155, miR-218 and miR-27B expression in ASCs. For all three miRNAs, higher expression in cells was significantly correlated with a better clinical outcome, as shown in **Figures 8 - 10****.** Whilst this correlative study does not investigate the role of miRNA in the functioning of injected ASCs, it is a very breakthrough. These results represent a novel prognostic miRNA signature that indicate the efficacy of ASC use in OA therapeutics.

## Claims

1. Use of a composition comprising at least the miRNA miR-218 for stimulating, *in vitro,* the immunosuppressive properties of multipotent stromal or mesenchymal stem cells, or MSCs.

2. Use according to claim 1, wherein said miR-218 comprises the nucleic acid sequence as set forth in SEQ ID NO : 1.

3. Use according to claim 1 or 2, wherein said composition comprises or consists essentially of at least the miRNAs miR-218, miR-155 and miR-27B.

4. Use according to anyone of claims 1 or 3, said composition comprising miR-218 comprising the nucleic acid sequence as set forth in SEQ ID NO : 1, miR-155 comprising the nucleic acid sequence as set forth in SEQ ID NO : 2 and miR-27B comprising the nucleic acid sequence as set forth in SEQ ID NO : 3.

5. Isolated immunosuppressive MSC having an expression level of at least the miRNA miR-218 of at least 1.3 fold higher compared the expression level of the miR-218 of a non-immunosuppressive MSC.

6. Isolated immunosuppressive MSC according to claim 5 having an expression level of at least the miRNAs miR-218, miR-155 and miR-27B respectively 1.3 fold higher, 1.5 fold higher and 1.8 fold higher compared to the expression of miRNAs miR-218, miR-155 and miR-27B respectively of a non immunosuppressive MSC.

7. Composition comprising, or comprising essentially, the miRNAs miR-218 comprising the nucleic acid sequence as set forth in SEQ ID NO: 1 miR-155 comprising the nucleic acid sequence as set forth in SEQ ID NO: 2 and miR-27B comprising the nucleic acid sequence as set forth in SEQ ID NO: 3.

8. Composition comprising at least one cell according to claim 5 or 6, for its use for the treatment of inflammatory pathologies or pathologies associated with inflammation.

9. Composition for its use according to claim 8, wherein said pathologies associated with an immunosuppression are chosen from the group consisting of: osteoarticular diseases, inflammatory and autocrine diseases.

10. Composition for its use according to claim 9, wherein said osteoarticular diseases are osteoarthritis, arthritis and related musculoskeletal disorders and sclerodermias.

11. Method for the *in vitro* stimulation of the immunosuppressive properties of isolated MSCs, said method comprising a step of stimulating the expression of at least the miRNA miR-218.

12. Method according to claim 11, wherein the step of stimulating the expression of at least the miRNA miR-218 is a step of ectopic expression of said at least miR-218.

13. Kit comprising :
- at least the miRNA miR-218 and
- at least one isolated MSC.

14. Kit according to claim 13 comprising :
- the miRNAs miR-218, miR-155 and miR-27B, and
- at least one isolated MSC.


SEQUENCE LISTING

<110>  Institut National de la Recherche Medicale
       Centre Hospitalier Universitaire de Montpellier
       Universite de Montpellier

<120>  Mesenchymal stem cells and their use

<130>  BR91690/RIM

<160>  9

<170>  PatentIn version 3.5

<210>  1
<211>  21
<212>  RNA
<213>  Artificial Sequence

<220>
<223>  mir-218

<400>  1
uugugcuuga ucuaaccaug u                                                21

<210>  2
<211>  23
<212>  RNA
<213>  Artificial Sequence

<220>
<223>  mir-155

<400>  2
uuaaugcuaa ucgugauagg ggu                                              23

<210>  3
<211>  21
<212>  RNA
<213>  Artificial Sequence

<220>
<223>  mir-27b

<400>  3
uucacagugg cuaaguucug c                                                21

<210>  4
<211>  110
<212>  RNA
<213>  Artificial Sequence

<220>
<223>  pre-mir-218

<400>  4
gugauaaugu agcgagauuu ucuguugugc uugaucuaac caugugguug cgagguauga      60

guaaaacaug guuccgucaa gcaccaugga acgucacgca gcuuucuaca                110

<210>  5
<211>  22
<212>  RNA
<213>  Artificial Sequence

<220>
<223>  mir-218 antisense

<400>  5
augguuccgu caagcaccau gg                                                22


<210>  6
<211>  65
<212>  RNA
<213>  Artificial Sequence

<220>
<223>  pre-mir-155

<400>  6
cuguuaaugc uaaucgugau agggguuuuu gccuccaacu gacuccuaca uauuagcauu      60

aacag                                                                   65


<210>  7
<211>  22
<212>  RNA
<213>  Artificial Sequence

<220>
<223>  antisense mir-155

<400>  7
cuccuacaua uuagcauuaa ca                                                22


<210>  8
<211>  97
<212>  RNA
<213>  Artificial Sequence

<220>
<223>  pre-mir-27b

<400>  8
accucucuaa caaggugcag agcuuagcug auuggugaac agugauuggu uuccgcuuug      60

uucacagugg cuaaguucug caccugaaga gaaggug                               97


<210>  9
<211>  28
<212>  RNA
<213>  Artificial Sequence

<220>
<223>  antisense mir27b

<400>  9
aggugcagag cuuagcugau uggugaac                                          28